# EUROPÄISCHE PATENTSCHRIFT

(11) **EP 3 559 191 B1**
(45) Veröffentlichungstag und Bekanntmachung des Hinweises auf die Patenterteilung: **17.08.2022**
(21) Anmeldenummer: 17800412.3
(22) Anmeldetag: 27.10.2017
(51) Int. Cl.: C11D 3/12, A61K 8/02, C11D 11/00, C11D 17/00

(54) **REINIGUNGSMITTEL MIT ABRASIVEN VULKANISCHEM GLAS**
CLEANING AGENT COMPRISING ABRASIVE VOLCANIC GLASS
PRODUIT DE NETTOYAGE COMPRENANT DU VERRE VOLCANIQUE ABRASIF

(30) Priorität: 21.12.2016 DE 102016225902
(43) Veröffentlichungstag der Anmeldung: 30.10.2019
(73) Patentinhaber: Henkel AG & Co. KGaA, 40589 Düsseldorf (DE)
(72) Erfinder: DREJA, Michael, 41469 Neuss (DE); BUISKER, Detlef, 45134 Essen (DE)
(86) Internationale Anmeldenummer: PCT/EP2017/077685
(87) Internationale Veröffentlichungsnummer: WO 2018/114100

(56) Entgegenhaltungen:
- WO-A2-2015/097228
- DE-A1- 2 539 733
- DE-A1- 2 739 776
- DE-A1- 2 843 517
- DE-B- 1 256 343
- DE-B- 1 289 600
- FR-A- 1 371 055
- GB-A- 717 589
- GB-A- 2 178 441
- US-A- 2 093 660
- US-A- 4 051 056
- US-A- 4 457 856
- AKOPYAN, G: "Composition containing expanded perlite for cleaning solid surfaces", CHEMICAL ABSTRACTS, CHEMICAL ABSTRACTS SERVICE (C A S), US, 9. Januar 1995 (1995-01-09), XP000666848, ISSN: 0009-2258

## Beschreibung

Die vorliegende Erfindung betrifft eine Zusammensetzung, die wenigstens ein Tensid, sowie wenigstens ein natürliches Material mit geringer Dichte bei gleichzeitig ausreichender Härte aufweist.

Die Reinigung insbesondere von harten Oberflächen, wie beispielsweise Böden oder auch Geschirr, stellt eine besondere Herausforderung dar, und zwar insbesondere dann, wenn hartnäckiger Schmutz auf diesen Oberflächen vorhanden ist. Reinigungsmittel, insbesondere Handgeschirrspülmittel, bedürfen zusätzlich zu der Reinigungsleistung durch tensidische und chemische Komponenten auch einer weiteren Leistungskomponente, die durch physikalische Wirkung entsteht, nämlich der Abrasivwirkung.

So beschreibt WO 2005/010138 A1 eine Zusammensetzung, die beispielsweise ein Pulver von Aprikosenkernen aufweist. Dieses ist zur Reinigung von harten Oberflächen als auch der menschlichen Haut geeignet. Unterschiedliche Abrasiva sind auch in US 4,457,856 A offenbart. Partikel von Schalen von Pistaziennüssen sind in Reinigungsmitteln enthalten, wie sie in EP 2 573 157 A1 beschrieben sind. Die gleichen abrasiven Partikel werden auch in WO 2013/043620 A1 beschrieben. Kunststoffe, harte Wachse oder Keramikteilchen werden beispielsweise in WO 2004/035720 A1 als abrasive Teilchen beschrieben. DE 25 39 733 A1, US 4,051,056 A, US 4,457,856 A und DE 27 39 776 A1 offenbaren Reinigungszusammensetzungen zum Reinigen von harten Oberflächen, die Bimsstein als Schleifmittel enthalten können.

Die bisher vorgeschlagenen Lösungen weisen jedoch Nachteile auf. Kunststoffpartikel werden üblicherweise aus Erdöl hergestellt und sind somit weder nachhaltig noch gut bioabbaubar. Hieraus entstehender Mikrokunststoff kann sich in der Umwelt anreichern. Die langfristigen Auswirkungen hierzu sind nicht geklärt. Zudem sind diese Partikel häufig rund. Je runder die Partikel sind, desto schlechter ist deren Abrasivwirkung.

Bekannt sind auch bioabbaubare Kunststoffe. Diese sind in der Regel jedoch nur unter bestimmten Bedingungen, wie einem hohen Druck oder einer hohen Temperatur bioabbaubar. Diese entsprechen jedoch nicht dem haushaltsüblichen Abwasserweg.

Die bei dem im Stand der Technik vorgeschlagenen natürlichen Produkte, wie Schalen oder Kerne von Obst, sind aus Umweltaspekten bevorzugt. Sie weisen jedoch üblicherweise eine geringe Härte auf, sodass die Reinigungswirkung, die hier zusätzlich erreicht werden soll, gering ist.

Aufgabe der vorliegenden Erfindung ist daher die Bereitstellung einer Zusammensetzung, die einerseits eine gute Abrasivwirkung aufweist, andererseits die Nachteile von Kunststoffpartikeln vermeidet. Überraschenderweise hat sich gezeigt, dass die der vorliegenden Erfindung zu Grunde liegende Aufgabe gelöst wird durch eine Zusammensetzung, die wenigstens ein Tensid sowie wenigstens ein natürliches Material aufweist, wobei das natürliche Material eine Mohshärte von 4 bis 6, insbesondere von 5 bis 6, aufweist, wobei das natürliche Material in Hydrogelpartikeln eingebettet vorliegt, wobei das natürliche Material Bimsstein ist, und die Zusammensetzung eine Zusammensetzung zur Reinigung von harten Oberflächen ist.

Durch den Einsatz solches natürlichen Materials kann die Verwendung von Kunststoffen vermieden werden. Durch die genannte Mohshärte wird eine ausreichende Abrasivwirkung ermöglicht. Gleichzeitig wird verhindert, dass empfindliche Materialien, wie beispielsweise Oberflächen aus Glas, wie sie beispielsweise in einem Ceranfeld vorkommen, zerkratzt werden. Auch bei der manuellen Reinigung beispielsweise von Trinkgläsern erfolgt keine Beschädigung des Materials.

"Abrasivstoff" und "natürliches Material" werden in der vorliegenden Anmeldung synonym verwendet.

Die Härte des natürlichen Materials nach der Mohshärteskala beträgt 4 bis 6, insbesondere 5 bis 6. Die Härtewerte nach Mohs lassen sich durch den Vergleich von mehreren Werkstoffen ermitteln.

Eine Mohshärte nach 1 weist beispielsweise Talk auf, welches mit dem Fingernagel schabbar ist. Diamant weist eine Mohshärte von 10 auf, dem maximalen Ende der Skala.

Eine Härte von 6 oder weniger, ist insbesondere bevorzugt, da hier sichergestellt werden kann, dass auch empfindliche Oberflächen, wie beispielsweise Ceranfelder in der Küche zwar von Schmutz befreit werden, nicht jedoch beschädigt werden. Eine Härte von weniger als 4 ist dabei nachteilig, da hier keine besonders gute abrasive Wirkung erzielt wird.

Vorzugsweise weist der Abrasivstoff eine Teilchengröße im Bereich von 0,1 µm bis 50.000 µm, insbesondere von 0,1 µm bis 40.000 µm, vorzugsweise vom 0,5 µm bis 25.000 µm, insbesondere von 1 µm bis 10.000 µm oder von 10.000 µm oder weniger, vorzugsweise von 5 µm bis 5.000 µm oder von 10 µm bis 1.000 µm auf. Der Abrasivstoff mit einer entsprechenden Teilchengröße wird vom Verbraucher einerseits optisch wahrgenommen. Gerade bei Handgeschirrspülmitteln ist dies von Verbrauchern von Interesse. Diese Teilchengröße ist jedoch auch besonders geeignet hartnäckige Verschmutzungen zu entfernen. Größere Teilchen würden bei Druck zerbrechen. Zudem können diese nicht unter die Ränder von Schmutz gelangen, und diesen so von den Oberflächen ablösen.

Das natürliche Material im Sinne der vorliegenden Erfindung ist ein solches Material, das auf natürlich vorkommenden Rohstoffen basiert. Natürlich vorkommende Rohstoffe sind dabei solche, die natürlichen Ursprungs sind, also in der Natur ursprünglich vorkommen. Das natürliche Material im Sinne der vorliegenden Erfindung ist dabei nachhaltig und vergrößert nicht den ökologischen Fußabdruck. Dabei wird unter dem ökologischem Fußabdruck die Fläche auf der Erde verstanden, die notwendig ist, den Lebensstil und Lebensstandard eines Menschen dauerhaft zu ermöglichen. Dies schließt Flächen ein, die zur Produktion von Kleidung und Nahrung oder zur Bereitstellung von Energie benötigt werden, aber auch zur Entsorgung von Müll. Eine physikalische Nachbereitung ist erfindungsgemäß nicht ausgeschlossen. Bevorzugt ist das natürliche Material, ein anorganisches Material, welches insbesondere biologisch neutral und frei von Schwermetallen ist beziehungsweise diese Schwermetalle nicht mobilisierbar sind.

Das natürliche Material weist vorzugsweise keine runde Form auf. Bestimmt man einen Partikelformfaktor, der das Seitenverhältnis von Partikeln zueinander definiert, so würde ein Wert von 1 für eine perfekte runde Form und ein Wert von 0 für eine Linearform stehen. Erfindungsgemäß weist der Abrasivstoff vorzugsweise einen Partikelformfaktor von 0,1 bis 0,97, insbesondere von 0,15 bis 0,9, insbesondere von 0,20 bis 0,80, vorzugsweise von 0,3 bis 0,70 oder bis 0,60 auf, insbesondere Werte von 0,30 oder 0,40 bis 0,50 sind bevorzugt. Partikel mit einem Partikelformfaktor von 1 würden kaum abrasive Wirkung aufweisen. Eine vollständige lineare Form würde hingegen nicht stabil sein, da entsprechende Stäbchen beim ausüben von Druck zerstört werden könnten. Die Zusammensetzung weist das wenigstens eine natürliche Material in einem Anteil von 0,02 Gew.-% bis 40 Gew.-%, insbesondere von 0,05 Gew.-% bis 30 Gew.-% oder bis 35 Gew.-%, vorzugsweise von 0,1 Gew.-% bis 20 Gew.-% oder bis 15 Gew.-%, besonders bevorzugt von 0,2 Gew.-% bis 5 Gew.-% auf. Dabei kann die Zusammensetzung ein natürliches Material oder Mischungen unterschiedlicher natürlicher Materialien aufweisen. Bevorzugt weist sie ein natürliches Material als Abrasivstoff auf.

Der Anteil des natürlichen Materials in der Zusammensetzung ist dabei Abhängig davon, um was es sich bei der Zusammensetzung handelt. Die Zusammensetzung ist ein Reinigungsmittel für harte Oberflächen, bevorzugt ein Reinigungsmittel zur manuellen Reinigung von harten Oberflächen, insbesondere ein Reinigungsmittel zur manuellen Reinigung von Geschirr, als ein Handgeschirrspülmittel. In diesem Fall beträgt der Anteil an Abrasivstoff vorzugsweise 0,03 Gew.-% bis 10 Gew.-%. Es ist jedoch auch möglich, dass die Zusammensetzung ein Reinigungsmittel für harte Oberflächen wie beispielsweise eine Scheuermilch ist. In diesem Fall beträgt der Anteil an Abrasivstoffen vorzugsweise 10 Gew.-% bis 40 Gew.-%, insbesondere 15 Gew.-% bis 30 Gew.-%.

Der Abrasivstoff ist Bimsstein, welcher auch als Bims bezeichnet wird. Bims oder Bimsstein ist ein poröses glasiges Vulkangestein, dessen Dichte aufgrund der zahlreichen Poren, die einen wesentlichen Teil des Volumens ausmachen, kleiner ist als die von Wasser, was bedeutet, dass Bims in Wasser schwimmt.

Es wurde festgestellt, dass Bimsstein sehr gute Abrasiveigenschaften aufweist und als Naturstoff eine hohe Umweltverträglichkeit aufweist. Ferner dient Bimsstein durch seine hohe Porosität als ausgezeichneter Träger für Duftstoffe und Farbstoffe.

In einer besonders bevorzugten Ausführungsform liegen die Abrasivstoffpartikel als Teil von zweiten Partikeln vor, wobei die zweiten Partikel, die auch als "beads" bezeichnet werden können, ein Hydrogel umfassen, wobei das Hydrogel eine Matrix für die Abrasivstoffpartikel bildet. Dadurch, dass das die Abrasivstoffe in Hydrogelpartikeln eingebettet vorliegen, können die Abrasivstoffpartikel gut dispergiert werden.

Dabei umfassen die zweiten Partikel den wenigstens einen Abrasivstoff bevorzugt in einer Menge von 2 Gew.-% bis 20 Gew.-%, und weiter bevorzugt 4 Gew.-% bis 18 Gew.-%, und weiter bevorzugt 5 Gew.-% bis 17 Gew.-%, und weiter bevorzugt 6 Gew.-% bis 16 Gew.-%, und weiter bevorzugt 8 Gew.-% bis 14 Gew.-%, und weiter bevorzugt 9 Gew.-% bis 11 Gew.-% bezogen auf die Masse der zweiten Partikel.

Dabei umfasst die Zusammensetzung die zweiten Partikel in einer durchschnittlichen Größe von 100 Mikrometer bis 2000 Mikrometer, und bevorzugt 206 Mikrometer bis 1894 Mikrometer, und weiter bevorzugt 311 Mikrometer bis 1789 Mikrometer, und weiter bevorzugt 417 Mikrometer bis 1683 Mikrometer, und weiter bevorzugt 522 Mikrometer bis 1578 Mikrometer, und weiter bevorzugt 628 Mikrometer bis 1472 Mikrometer, und weiter bevorzugt 733 Mikrometer bis 1367 Mikrometer, und weiter bevorzugt 839 Mikrometer bis 1261 Mikrometer, und weiter bevorzugt 944 Mikrometer bis 1156 Mikrometer.

Dabei umfasst die Martrix der zweiten Partikel, in der die Abrasivstoffpartikel eingelagert sind, bevorzugt Carrageenan und/oder Agar. Weiter bevorzugt umfasst die Martrix Carrageenan und/oder Agar in einer Menge von >80 Gew.-% bezogen auf die Masse der zweiten Partikel.

Ferner umfassen die zweiten Partikel (Farb)pigmente in einer Menge von <5 Gew.-% bevorzugt < 1 Gew-% bezogen auf die Masse der zweiten Partikel.

Bevorzugt weist die Zusammensetzung wenigsten ein Polyacrylsäurepolymer auf. Bevorzugt ist das Polyacrylsäurepolymer ein alkali-quellbares emulsionsbildendes Polymer. Der Begriff "Polyacrylsäurepolymer" ist im Zusammenhang mit der vorliegenden Erfindung dahingehend aufzufassen, dass hierunter auch Copolymere mit anderen Monomereinheiten als Acrylsäure fallen, wobei der Acrylsäureanteil bevorzugt > 10 Gew.-% bezogen auf das Gesamtgewicht des Polymers ist, weiter bevorzugt > 20 Gew.-%. Der Begriff "Acrylsäure" ist im Zusammenhang mit der vorliegenden Anmeldung dahingehend aufzufassen, dass hierunter auch Derivate der Acrylsäure fallen.

Dabei umfasst die Zusammensetzung das Polyacrylsäurepolymer in einer Menge von 0,1 Gew.-% bis 4 Gew.-%, und bevorzugt 1 Gew.-% bis 2,5 Gew.-%, und weiter bevorzugt 1,5 Gew.-% bis 2,5 Gew.-%,

Der Abrasivstoff ist Bimsstein.

Es wurde festgestellt, dass Bimsstein sehr gute Abrasiveigenschaften und als Naturstoff eine hohe Umweltverträglichkeit aufweist. Ferner dient Bimsstein durch seine hohe Porosität als Träger für Duftstoffe und Farbstoffe. Allerdings schwimmt Bimsstein wegen seiner hohen Porosität und geringen Dichte in wässrigen Lösungen nachteiligerweise an der Oberfläche, was insbesondere der Verwendung als Reinigungsmittel entgegensteht.

Es wurde nun überraschend gefunden, dass sich in einer wässrigen Zusammensetzung, die Abrasivpartikel aus Bimsstein und ein Polyacrylsäurepolymer umfasst, die Partikel umfassend den Abrasivstoff homogen suspendieren lassen, ohne dass diese an der Oberfläche der Zusammensetzung schwimmen oder bei Durchmischung Aggregieren. Eine solche Zusammensetzung kann vorteilhafterweise als Reinigungsmittel, insbesondere Handgeschirrspülmittel verwendet werden.

Die erfindungsgemäße Zusammensetzung weist das wenigstens eine Tensid in einem Anteil von 5 Gew.-% bis 35 Gew.-%, insbesondere von 8 Gew.-% bis 30 Gew.-%, vorzugsweise von 10 Gew.-% bis 25 Gew.-% auf. Gemeint ist somit der Gesamttensidgehalt. Wenigstens ein Tensid im Sinne der vorliegenden Erfindung bedeutet, dass die Zusammensetzung ein Tensid oder Mischungen mehrerer unterschiedlicher Tenside, wie beispielsweise anionischer Tenside (Aniontensid) und nichtionischer Tenside und amphoterer Tenside aufweisen kann. Besonders bevorzugt weist die Zusammensetzung ein Aniontensid und ein nichtionisches Tensid und ein amphoteres Tensid auf, insbesondere wenigstens ein Aniontensid, wenigstens ein nichtionisches Tensid und wenigstens ein amphoteres Tensid.

Anionische Tenside gemäß der Erfindung können aliphatische Sulfate wie Fettalkoholsulfate, Fettalkoholethersulfate, Dialkylethersulfate, Monoglyceridsulfate und aliphatische Sulfonate wie Alkansulfonate, Olefinsulfonate, Ethersulfonate, n-Alkylethersulfonate, Estersulfonate und Ligninsulfonate sein. Ebenfalls im Rahmen der vorliegenden Erfindung verwendbar sind Alkylbenzolsulfonate, Fettsäurecyanamide, Sulfobernsteinsäureester, Fettsäureisethionate, Acylaminoalkansulfonate (Fettsäuretauride), Fettsäuresarcosinate, Ethercarbonsäuren und Alkyl(ether)phosphate.

Die Alkylethersulfate, Alkyl- und/oder Arylsulfonate und/oder Alkylsulfate sowie die weiteren anionische Tenside werden üblicherweise als Alkalimetall-, Erdalkalimetall- und/oder Mono-, Dibeziehungsweise Trialkanolammoniumsalz und/oder aber auch in Form ihrer mit dem entsprechenden Afkalimetallhydroxid, Erdalkalimetallhydroxid und/oder Mono-, Di- beziehungsweise Trialkanolamin *in situ* zu neutralisierenden korrespondierenden Säure eingesetzt. Bevorzugt sind hierbei als Alkalimetalle Kalium und insbesondere Natrium, als Erdalkalimetalle Calcium und insbesondere Magnesium, sowie als Alkanolamine Mono-, Di- oder Triethanolamin. Besonders bevorzugt sind die Natriumsalze.

Alkylethersulfate (Fettalkoholethersulfate, *INCI* Alkyl Ether Sulfates) sind Produkte von Sulfatierreaktionen an alkoxylierten Alkoholen. Dabei versteht der Fachmann allgemein unter alkoxylierten Alkoholen die Reaktionsprodukte von Alkylenoxid, bevorzugt Ethylenoxid, mit Alkoholen, im Sinne der vorliegenden Erfindung bevorzugt mit längerkettigen Alkoholen, das heißt mit aliphatischen geradkettigen oder ein oder mehrfach verzweigten, acyclischen oder cyclischen, gesättigten oder ein oder mehrfach ungesättigten, vorzugsweise geradkettigen, acyclischen, gesättigten, Alkoholen mit 6 bis 22, vorzugsweise 8 bis 18, insbesondere 10 bis 16 und besonders bevorzugt 12 bis 14 Kohlenstoffatomen. In der Regel entsteht aus n Molen Ethylenoxid und einem Mol Alkohol, abhängig von den Reaktionsbedingungen, ein komplexes Gemisch von Additionsprodukten unterschiedlicher Ethoxylierungsgrade (n = 1 bis 30, vorzugsweise 0,30 bis 20, insbesondere 0,30 bis 10, besonders bevorzugt 0,30 bis 5). Eine weitere Ausführungsform der Alkoxylierung besteht im Einsatz von Gemischen der Alkylenoxide, bevorzugt des Gemisches aus Ethylenoxid und Propylenoxid. Ganz besonders bevorzugt im Sinne der vorliegenden Erfindung sind niederethoxylierte Fettalkohole mit 0,30 bis 4 Ethylenoxideinheiten (EO), insbesondere 0,30 bis 2 EO, beispielsweise 0,50 EO, 1,0 EO, 1,3 EO und/oder 2,0 EO wie Na-C₁₂₋₁₄-Fettalkohol+0,5EO-sulfat, Na-C₁₂₋₄₄-Fettalkohot+1,3EO-sutfat, Na-C₁₂₋₁₄-Fettalkohol+2,0EO-sulfat und/oder Mg-C₁₁₋₁₄-Fettalkohol+1,0EO-sulfat.

Das erfindungsgemäße Mittel kann ein oder mehrere Alkylethersulfate in einer Menge von üblicherweise 1 bis 40 Gew.-%, vorzugsweise 3 bis 30 Gew.-%, insbesondere mehr als 6 bis 26 Gew.-%, besonders bevorzugt 8 bis 20 Gew.-%, äußerst bevorzugt 10 bis 16 Gew.-% enthalten.

Die Alkylsulfonate *(INCI* Sulfonic Acids) weisen üblicherweise einen aliphatischen gerad-kettigen oder ein- oder mehrfach verzweigten, acyclischen oder cyclischen, gesättigten oder ein- oder mehrfach ungesättigten, vorzugsweise verzweigten, acyclischen, gesättigten, Alkylrest mit 6 bis 22, vorzugsweise 9 bis 20, insbesondere 11 bis 18 und besonders bevorzugt 13 bis 17 Kohlenstoffatomen auf.

Geeignete Alkylsulfonate sind dementsprechend die gesättigten Alkansulfonate, die ungesättigten Olefinsulfonate und die - sich formal von den auch den Alkylethersulfaten zugrundeliegenden alkoxylierten Alkoholen ableitenden - Ethersulfonate, bei denen man endständige Ethersulfonate (n-Ethersulfonate) mit an die Polyether-Kette gebundener Sulfonat-Funktion und innenständige Ethersulfonate (i-Ethersulfonate) mit dem Alkylrest verknüpfter Sulfonat-Funktion. Erfindungsgemäß bevorzugt sind die Alkansulfonate, insbesondere Alkansulfonate mit einem verzweigten, vorzugsweise sekundären, Alkylrest, beispielsweise das sekundäre Alkansulfonat sek. Na-C₁₃₋₁₇-Alkansulfonat *(INCI* Sodium C₁₄₋₁₇ Alkyl Sec Sulfonate).

Bevorzugt eingesetzte Arylsulfonate sind Alkylbenzolsulfonate, wobei die Alkylreste verzweigte und unverzweigte Ketten mit C₁-C₂₀, bevorzugt C2-C18, besonders bevorzugt C6-C16 und am meisten bevorzugt C₈-C₁₂ darstellen. Besonders bevorzugte Beispiele sind hierbei LAS und/oder Cumolsulfonat.

Das erfindungsgemäße Mittel kann ein oder mehrere Alkyl- und/oder Arylsulfonate in einer Menge von üblicherweise 0,1 bis weniger als 40 Gew.-%, vorzugsweise 0,1 bis 30 Gew.-%, insbesondere 1 bis weniger als 14 Gew.-%, besonders bevorzugt 2 bis 10 Gew.-%, äußerst bevorzugt 4 bis 8 Gew.-% enthalten.

In der vorliegenden Erfindung können auch Alkylsulfate wie zum Beispiel Fettalkoholsulfate eingesetzt werden. Geeignete Alkylsulfate sind Sulfate gesättigter und ungesättigter Fettalkohole mit C₆-C22, bevorzugt C₁₀-C₁₈ und besonders bevorzugt von C11-C16. Besonders geeignete Alkylsulfate sind solche mit nativem C-Schnitt C12-14-16 und/oder petrochemischem C-Schnitt C₁₂-₁₃, C₁₄-C₁₅ im Bereich von 0 bis 15 %, bevorzugt 0-10%, besonders bevorzugt 0 bis 8 %.

Das erfindungsgemäße Mittel kann zusätzlich ein oder mehrere weitere anionische Tenside enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Geeignete weitere anionische Tenside sind insbesondere aliphatische Sulfate wie Monoglyceridsulfate sowie Estersulfonate (Sulfofettsäureester), Ligninsulfonate, Fettsäurecyanamide, anionische Sulfobernsteinsäuretenside, Fettsäureisethionate, Acylaminoalkansul-fonate (Fettsäuretauride), Fettsäuresarcosinate, Ethercarbonsäuren und Alkyl(ether)phosphate.

Geeignete weitere anionische Tenside sind auch anionische Gemini-Tenside mit einer Diphenyloxid-Grundstruktur, 2 Sulfonatgruppen und einem Alkylrest an einem oder beiden Benzolringen gemäß der Formel ⁻O₃S(C₆H₃R)O(C₆H₃R')SO₃⁻, in der R für einen Alkylrest mit beispielsweise 6, 10, 12 oder 16 Kohlenstoffatomen und R' für R oder H steht (Dowfax^{®} Dry Hydrotrope Powder mit C₁₆-Alkylrest(en); *INCI* Sodium Hexyldiphenyl Ether Sulfonate, Disodium Decyl Phenyl Ether Disulfonate, Disodium Lauryl Phenyl Ether Disulfonate, Disodium Cetyl Phenyl Ether Disulfonate) und fluorierte anionische Tenside, insbesondere perfluorierte Alkylsulfonate wie Ammonium-C_{9/10}-Perfluoroalkylsulfonat (Fluorad^{®} FC 120) und Perfluoroctansulfonsäure-Kalium-Salz (Fluorad^{®} FC 95).

Besonders bevorzugte weitere anionische Tenside sind die anionischen Sulfobernsteinsäuretenside Sulfosuccinate, Sulfosuccinamate und Sulfosuccinamide, insbesondere Sulfosuccinate und Sulfosuccinamate, äußerst bevorzugt Sulfosuccinate. Bei den Sulfosuccinaten handelt es sich um die Salze der Mono- und Diester der Sulfobernsteinsäure HOOCCH(SO₃H)CH₂COOH, während man unter den Sulfosuccinamaten die Salze der Monoamide der Sulfobernsteinsäure und unter den Sulfosuccinamiden die Salze der Diamide der Sulfobernsteinsäure versteht. Eine ausführliche Beschreibung dieser bekannten Aniontenside liefern A. Domsch und B. Irrgang in Anionic surfactants: organic chemistry (edited by H. W. Stache; Surfactant science series; volume 56; ISBN 0-82479394-3; Marcel Dekker, Inc., New York 1996, S. 501-549).

Bei den Salzen handelt es sich bevorzugt um Alkalimetallsalze, Ammoniumsalze sowie Mono-, Dibeziehungsweise Trialkanolammoniumsalze, beispielsweise Mono-, Di- beziehungsweise Triethanolammoniumsalze, insbesondere um Lithium-, Natrium-, Kalium- oder Ammoniumsalze, besonders bevorzugt Natrium- oder Ammoniumsalze, äußerst bevorzugt Natriumsalze.

In den Sulfosuccinaten ist eine beziehungsweise sind beide Carboxylgruppen der Sulfobernsteinsäure vorzugsweise mit einem beziehungsweise zwei gleichen oder verschiedenen unverzweigten oder verzweigten, gesättigten oder ungesättigten, acyclischen oder cyclischen, optional alkoxylierten Alkoholen mit 4 bis 22, vorzugsweise 6 bis 20, insbesondere 8 bis 18, besonders bevorzugt 10 bis 16, äußerst bevorzugt 12 bis 14 Kohlenstoffatomen verestert. Besonders bevorzugt sind die Ester unverzweigter und/oder gesättigter und/oder acyclischer und/-oder alkoxylierter Alkohole, insbesondere unverzweigter, gesättigter Fettalkohole und/-oder unverzweigter, gesättigter, mit Ethylen- und/oder Propylenoxid, vorzugsweise Ethylenoxid, alkoxylierter Fettalkohole mit einem Alkoxylierungsgrad von 1 bis 20, vorzugsweise 1 bis 15, insbesondere 1 bis 10, besonders bevorzugt 1 bis 6, äußerst bevorzugt 1 bis 4. Die Monoester werden im Rahmen der vorliegenden Erfindung gegenüber den Diestern bevorzugt. Ein besonders bevorzugtes Sulfosuccinat ist Sulfobernsteinsäurelaurylpolyglykolester-di-Natrium-Salz (Lauryl-EOsulfosuccinat, Di-Na-Salz; INCI Disodium Laureth Sulfosuccinate), das beispielsweise als *Tego*^{®} *Sulfosuccinat F 30 (Goldschmidt)* mit einem Sulfosuccinatgehalt von 30 Gew.-% kommerziell erhältlich ist.

In den Sulfosuccinamaten beziehungsweise Sulfosuccinamiden bildet eine beziehungsweise bilden beide Carboxyl-gruppen der Sulfobernsteinsäure vorzugsweise mit einem primären oder sekundären Amin, das einen oder zwei gleiche oder verschiedene, unverzweigte oder verzweigte, gesättigte oder ungesättigte, acyclische oder cyclische, optional alkoxylierte Alkylreste mit 4 bis 22, vorzugsweise 6 bis 20, insbesondere 8 bis 18, besonders bevorzugt 10 bis 16, äußerst bevorzugt 12 bis 14 Kohlenstoffatomen trägt, ein Carbonsäureamid. Besonders bevorzugt sind unverzweigte und/oder gesättigte und/oder acyclische Alkylreste, insbesondere unverzweigte, gesättigte Fettalkylreste. Weiterhin geeignet sind beispielsweise die folgenden gemäß INCI bezeichneten Sulfosuccinate und Sulfosuccinamate, die im *International Cosmetic Ingredient Dictionary and Handbook* näher beschrieben sind: Ammonium Dinonyl Sulfosuccinate, Ammonium Lauryl Sulfosuccinate, Diammonium Dimethicone Copolyol Sulfosuccinate, Diammonium Lauramido-MEA Sulfosuccinate, Diammonium Lauryl Sulfosuccinate, Diammonium Oleamido PEG-2 Sulfosuccinate, Diamyl Sodium Sulfosuccinate, Dicapryl Sodium Sulfosuccinate, Dicyclohexyl Sodium Sulfosuccinate, Diheptyl Sodium Sulfosuccinate, Dihexyl Sodium Sulfosuccinate, Diisobutyl Sodium Sulfosuccinate, Dioctyl Sodium Sulfosuccinate, Disodium Cetearyl Sulfosuccinate, Disodium Cocamido MEA-Sulfosuccinate, Disodium Cocamido MIPA-Sulfosuccinate, Disodium Cocamido PEG-3 Sulfosuccinate, Disodium Coco-Glucoside Sulfosuccinate, Disodium Cocoyl Butyl Gluceth-10 Sulfosuccinate, Disodium C12-15 Pareth Sulfosuccinate, Disodium Deceth-5 Sulfosuccinate, Disodium Deceth-6 Sulfosuccinate, Disodium Dihydroxyethyl Sulfosuccinylundecylenate, Disodium Dimethicone Copolyol Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Lauramido PEG-5 Sulfosuccinate, Disodium Lau-reth-6 Sulfosuccinate, Disodium Laureth-9 Sulfosuccinate, Disodium Laureth-12 Sulfosuccinate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocoamido MIPA-Sulfosuccinate, Disodium PEG-5 Laurylcitrate Sulfosuccinate, Disodium PEG-8 Palm Glycerides Sulfosuccinate, Disodium Ricinoleamido MEA-Sulfosuccinate, Disodium Sitostereth-14 Sulfosuccinate, Disodium Stearamido MEA-Sulfosuccinate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Di-TEA-Oleamido PEG-2 Sulfosuccinate, Ditridecyl Sodium Sulfosuccinate, Sodium Bisglycol Ricinosulfosuccinate, Sodium/MEA Laureth-2 Sulfosuccinate und Tetrasodium Dicarboxyethyl Stearyl Sulfosuccinamate. Noch ein weiteres geeignetes Sulfosuccinamat ist Dinatrium-C₁₆₋₁₈-alkoxypropylensulfosuccinamat.

Bevorzugte anionische Sulfobernsteinsäuretenside sind Imidosuccinat, Mono-Na-sulfobernsteinsäure-diisobutylester (Monawet^{®} MB 45), Mono-Na-sulfobernsteinsäure-di-octylester (Monawet^{®} MO-84 R2W, Rewopol^{®} SB DO 75), Mono-Na-sulfobernsteinsäure-di-tridecylester (Monawet^{®} MT 70), Fettalkoholpolyglykolsulfosuccinat-Na-NH₄-Salz (Sulfo-succinat S-2), Di-Na-sulfobernsteinsäure-mono-C_{12/14}-3EO-ester (Texapon^{®} SB-3), Natriumsulfobernsteinsäurediisooctylester (Texin^{®} DOS 75) und Di-Na-Sulfobernsteinsäure-mono-C_{12/18}-ester (Texin^{®} 128-P), insbesondere Mono-Na-sulfobernsteinsäure-dioctylester.

In einer besonderen Ausführungsform enthält das erfindungsgemäße Mittel als anionische Sulfobernsteinsäuretenside ein oder mehrere Sulfosuccinate, Sulfosuccinamate und/oder Sulfosuccinamide, vorzugsweise Sulfosuccinate und/oder Sulfosuccinamate, insbesondere Sulfosuccinate, in einer Menge von üblicherweise 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Zu den Amphotensiden (amphoteren Tensiden, zwitterionischen Tensiden), die erfindungsgemäß eingesetzt werden können, zählen Betaine, Alkylamidoalkylamine, alkylsubstituierte Aminosäuren, acylierte Aminosäuren beziehungsweise Biotenside, von denen die Betaine im Rahmen der erfindungsgemäßen Lehre bevorzugt werden.

Das erfindungsgemäße Mittel kann ein oder mehrere Amphotenside in einer Menge von üblicherweise 0,1 bis 20 Gew.-%, vorzugsweise 1 bis 15 Gew.-%, insbesondere 2 bis 12 Gew.-%, besonders bevorzugt 3 bis 10 Gew.-%, äußerst bevorzugt 4 bis 8 Gew.-% enthalten.

Geeignete Betaine sind die Alkylbetaine, die Alkylamidobetaine, die Imidazoliniumbetaine, die Sulfobetaine *(INCI* Sultaines) sowie die Phosphobetaine und genügen vorzugsweise Formel I,

R¹-[CO-X-(CH₂)ₙ]ₓ-N⁺(R²)(R³)-(CH₂)ₘ₋[CH(OH)-CH₂]_{y}-Y⁻ (I)

- in der R¹: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
X NH, NR⁴ mit dem C₁₋₄-Alkylrest R⁴, O oder S,
n eine Zahl von 1 bis 10, vorzugsweise 2 bis 5, insbesondere 3,
x 0 oder 1, vorzugsweise 1,
R², R³ unabhängig voneinander ein C₁₋₄-Alkylrest, gegebenenfalls hydroxysubstituiert wie zum Beispiel ein Hydroxyethylrest, insbesondere aber ein Methylrest,
m eine Zahl von 1 bis 4, insbesondere 1, 2 oder 3,
y 0 oder 1 und
Y COO, SO₃, OPO(OR⁵)O oder P(O)(OR⁵)O, wobei R⁵ ein Wasserstoffatom H oder ein C₁₄-Alkylrest
ist. Die Alkyl- und Alkylamidobetaine, Betaine der Formel (I) mit einer Carboxylatgruppe (Y⁻ = COO⁻), heißen auch Carbobetaine.

Bevorzugte Amphotenside sind die Alkylbetaine der Formel (la), die Alkylamidobetaine der Formel (Ib), die Sulfobetaine der Formel (Ic) und die Amidosulfobetaine der Formel (Id),

R¹-N⁺(CH₃)₂-CH₂COO⁻ (la)

R¹-CO-NH-(CH₂)₃-N⁺(CH₃)₂-CH₂COO⁻ (Ib)

R¹+N⁺(CH₃)₂-CH₂CH(OH)CH₂SO₃⁻ (Ic)

R¹⁻CO-NH-(CH₂)₃-N⁺(CH₃)₂-CH₂CH(OH)CH₂SO₃⁻ (Id)

in denen R¹ die gleiche Bedeutung wie in Formel (I) hat.

Besonders bevorzugte Amphotenside sind die Carbobetaine, insbesondere die Carbobetaine der Formel (la) und (Ib), äußerst bevorzugt die Alkylamidobetaine der Formel (Ib).

Beispiele geeigneter Betaine und Sulfobetaine sind die folgenden gemäß *INCI* benannten Verbindungen: Almondamidopropyl Betaine, Apricotamidopropyl Betaine, Avocadamidopropyl Betaine, Babassuamidopropyl Betaine, Behenamidopropyl Betaine, Behenyl Betaine, Betaine, Canolamidopropyl Betaine, Capryl/Capramidopropyl Betaine, Carnitine, Cetyl Betaine, Cocamidoethyl Betaine, Cocamidopropyl Betaine, Cocamidopropyl Hydroxysultaine, Coco-Betaine, Coco-Hydroxysultaine, Coco/Oleamidopropyl Betaine, Coco-Sultaine, Decyl Betaine, Dihydroxyethyl Oleyl Glycinate, Dihydroxyethyl Soy Glycinate, Di-hydroxyethyl Stearyl Glycinate, Dihydroxyethyl Tallow Glycinate, Dimethicone Propyl PG-Betaine, Erucamidopropyl Hydroxysultaine, Hydrogenated Tallow Betaine, Isostearamidopropyl Betaine, Lauramidopropyl Betaine, Lauryl Betaine, Lauryl Hydroxysultaine, Lauryl Sultaine, Milkamidopropyl Betaine, Minkamidopropyl Betaine, Myristamidopropyl Betaine, Myristyl Betaine, Oleamidopropyl Betaine, Oleamidopropyl Hydroxysultaine, Oleyl Betaine, Olivamidopropyl Betaine, Palmamidopropyl Betaine, Palmitamidopropyl Be-taine, Palmitoyl Carnitine, Palm Kernelamidopropyl Betaine, Polytetrafluoroethylene Acetoxypropyl Betaine, Ricinoleamidopropyl Betaine, Sesamidopropyl Betaine, Soyamidopropyl Betaine, Stearamidopropyl Betaine, Stearyl Betaine, Tallowamidopropyl Betaine, Tallowamidopropyl Hydroxysultaine, Tallow Betaine, Tallow Dihydroxyethyl Betaine, Undecylenamidopropyl Betaine und Wheat Germamidopropyl Betaine. Ein bevorzugtes Betain ist beispielsweise Cocamidopropyl Betaine (Cocoamidopropylbetain).

Die Alkylamidoalkylamine *(INCI* Alkylamido Alkylamines) sind Amphotenside der Formel (III),

R⁹-CO-NR¹⁰-(CH₂)ᵢ-N(R¹¹)-(CH₂CH₂O)ⱼ(CH₂)ₖ-[CH(OH)]ₗ-CH₂-Z-OM (III)

in der
- R⁹: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁰: ein Wasserstoffatom H oder ein C₁₄-Alkylrest, vorzugsweise H,
- i: eine Zahl von 1 bis 10, vorzugsweise 2 bis 5, insbesondere 2 oder 3,
- R¹¹: ein Wasserstoffatom H oder CH₂COOM (zu M s.u.),
- j: eine Zahl von 1 bis 4, vorzugsweise 1 oder 2, insbesondere 1,
- k: eine Zahl von 0 bis 4, vorzugsweise 0 oder 1,
- I: 0 oder 1, wobei k = 1 ist, wenn I = 1 ist,
- Z: CO, SO₂, OPO(OR¹²) oder P(O)(OR¹²), wobei R¹² ein C₁₋₄-Alkylrest oder M (s.u.) ist, und
- M: ein Wasserstoff, ein Alkalimetall, ein Erdalkalimetall oder ein protoniertes Alkanolamin, zum Beispiel protoniertes Mono-, Di- oder Triethanolamin, ist.

Bevorzugte Vertreter genügen den Formeln lila bis IIId,

R⁹-CO-NH-(CH₂)₂-N(R¹¹)-CH₂CH₂O-CH₂-COOM (IIIa)

R⁹-CO-NH-(CH₂)₂-N(R¹¹)-CH₂CH₂O-CH₂CH₂-COOM (IIIb)

R⁹-CO-NH-(CH₂)₂-N(R¹¹)-CH₂CH₂O-CH₂CH(OH)CH₂-SO₃M (IIIc)

R⁹-CO-NH-(CH₂)₂-N(R¹¹)-CH₂CH₂O-CH₂CH(OH)CH₂-OPO₃HM (IIId)

in denen R⁹, R¹¹ und M die gleiche Bedeutung wie in Formel (III) haben.

Beispielhafte Alkylamidoalkylamine sind die Folgenden gemäß *INCI* benannten Verbindungen: Cocoamphodipropionic Acid, Cocobetainamido Amphopropionate, DEA-Cocoamphodipropionate, Disodium Caproamphodiacetate, Disodium Caproamphodipropionate, Disodium Capryloamphodiacetate, Disodium Capryloamphodipropionate, Disodium Cocoamphocarboxyethylhydroxypropylsulfonate, Disodium Cocoamphodiacetate, Disodium Cocoamphodipropionate, Disodium Isostearoamphodiacetate, Disodium Isostearoamphodipropionate, Disodium Laureth-5 Carboxyamphodiacetate, Disodium Lauroamphodiacetate, Disodium Lauroamphodipropionate, Disodium Oleoamphodipropionate, Disodium PPG-2-lsodeceth-7-Carboxyamphodiacetate, Disodium Stearoamphodiacetate, Disodium Tallowamphodiacetate, Disodium Wheatgermamphodiacetate, Lauroamphodipropionic Acid, Quaternium-85, Sodium Caproamphoacetate, Sodium Caproamphohydroxypropylsulfonate, Sodium Caproamphopropionate, Sodium Capryloamphoacetate, Sodium Capryloamphohydroxypropylsulfonate, Sodium Capryioamphopropionate, Sodium Cocoamphoacetate, Sodium Cocoamphohydroxypropylsulfonate, Sodium Cocoamphopropionate, Sodium Cornamphopropionate, Sodium Isostearoamphoacetate, Sodium Isostearoamphopropionate, Sodium Lauroamphoacetate, Sodium Lauroamphohydroxypropylsulfonate, Sodium Lauroampho PG-Acetate Phosphate, Sodium Lauroamphopropionate, Sodium Myristoamphoacetate, Sodium Oleoamphoacetate, Sodium Oleoamphohydroxypropylsulfonate, Sodium Oleoamphopropionate, Sodium Ricinoleoamphoacetate, Sodium Stearoamphoacetate, Sodium Stearoamphohydroxypropylsulfonate, Sodium Stearoamphopropionate, Sodium Tallamphopropionate, Sodium Tallowamphoacetate, Sodium Undecylenoamphoacetate, Sodium Undecylenoamphopropionate, Sodium Wheat Germamphoacetate und Trisodium Lauroampho PG-Acetate Chloride Phosphate.

Erfindungsgemäß bevorzugte alkylsubstituierte Aminosäuren *(INCI* Alkyl-Substituted Amino Acids) sind monoalkylsubstituierte Aminosäuren gemäß Formel (IV),

R¹³-NH-CH(R¹⁴)-(CH₂)ᵤ-COOM' (IV)

in der
- R¹³: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁴: ein Wasserstoffatom H oder ein C₁₋₄-Alkylrest, vorzugsweise H,
- u: eine Zahl von 0 bis 4, vorzugsweise 0 oder 1, insbesondere 1, und
- M': ein Wasserstoff, ein Alkalimetall, ein Erdalkalimetall oder ein protoniertes Alkanolamin, zum Beispiel protoniertes Mono-, Di- oder Triethanolamin, ist,
alkylsubstituierte Iminosäuren gemäß Formel (V),

R¹⁵-N-[(CH₂)ᵥ-COOM"]₂ (V)

- in der R¹⁵: ein gesättigter oder ungesättigter C₆₋₂₂-Alkylrest, vorzugsweise C₈₋₁₈-Alkyl-rest, insbesondere ein gesättigter C₁₀₋₁₆-Alkylrest, beispielsweise ein gesättigter C₁₂₋₁₄-Alkylrest,
- v: eine Zahl von 1 bis 5, vorzugsweise 2 oder 3, insbesondere 2, und
- M": ein Wasserstoff, ein Alkalimetall, ein Erdalkalimetall oder ein protoniertes Alkanolamin, zum Beispiel protoniertes Mono-, Di- oder Triethanolamin, wobei M" in den beiden Carboxygruppen die gleiche oder zwei verschiedene Bedeutungen haben kann, zum Beispiel Wasserstoff und Natrium oder zweimal Natrium sein kann, ist,
und mono- oder dialkylsubstituierte natürliche Aminosäuren gemäß Formel (VI),

R¹⁶-N(R¹⁷)-CH(R¹⁸)COOM‴ (VI)

in der
- R¹⁶: ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest,
- R¹⁷: ein Wasserstoffatom oder *ein* C₁₄-Alkylrest, gegebenenfalls hydroxy- oder aminsubstituiert, zum Beispiel ein Methyl-, Ethyl-, Hydroxyethyl- oder Aminpropylrest,
- R¹⁸: den Rest einer der 20 natürlichen α-Aminosäuren H₂NCH(R¹⁸)COOH, und
- M‴: ein Wasserstoff, ein Alkalimetall, ein Erdalkalimetall oder ein protoniertes Alkanolamin, zum Beispiel protoniertes Mono-, Di- oder Triethanolamin, ist.

Besonders bevorzugte alkylsubstituierte Aminosäuren sind die Aminopropionate gemäß Formel (IVa),

R¹³-NH-CH₂CH₂COOM' (IVa)

in der R¹³ und M' die gleiche Bedeutung wie in Formel (IV) haben.

Beispielhafte alkylsubstituierte Aminosäuren sind die folgenden gemäß *INCI* benannten Verbindungen: Aminopropyl Laurylglutamine, Cocaminobutyric Acid, Cocaminopropionic Acid, DEA-Lauraminopropionate, Disodium Cocaminopropyl Iminodiacetate, Disodium Dicarboxyethyl Cocopropylenediamine, Disodium Lauriminodipropionate, Disodium Steariminodipropionate, Disodium Tallowiminodipropionate, Lauraminopropionic Acid, Lauryl Aminopropylglycine, Lauryl Diethylenediaminoglycine, Myristaminopropionic Acid, Sodium C12-15 Alkoxypropyl Iminodipropionate, Sodium Cocaminopropionate, Sodium Lauraminopropionate, Sodium Lauriminodipropionate, Sodium Lauroyl Methylaminopropionate, TEA-Lauraminopropionate und TEA-Myristaminopropionate.

Acylierte Aminosäuren sind Aminosäuren, insbesondere die 20 natürlichen α-Aminosäuren, die am Aminostickstoffatom den Acylrest R¹⁹CO einer gesättigten oder ungesättigten Fettsäure R¹⁹COOH tragen, wobei R¹⁹ ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest ist. Die acylierten Aminosäuren können auch als Alkalimetallsalz, Erdalkalimetallsalz oder Alkanolammoniumsalz, zum Beispiel Mono-, Di- oder Triethanolammoniumsalz, eingesetzt werden. Beispielhafte acylierte Aminosäuren sind die gemäß *INCI* unter Amino Acids zusammengefaßten Acylderivate, zum Beispiel Sodium Cocoyl Glutamate, Lauroyl Glutamic Acid, Capryloyl Glycine oder Myristoyl Methylalanine.

Besonders bevorzugte Amphotenside sind Biotenside. Biotenside sind Tensidmoleküle, die auf der Basis nachwachsender Rohstoffe, beispielsweise aus Pflanzenöl- und Zuckersubstraten, hergestellt werden können.

Bevorzugte Biotenside sind Glycolipide, welche beispielsweise unter dem Handelnamen Sophoclean^{®} (Firma Givandan) oder Rewoferm^{®}SL 446 (Firma Evonik) kommerziell erhältlich sind. Vorzugsweise umfasst das Glycolipid ein Rhamnolipid und/oder ein Sophorolipid. Bevorzugt sind weiterhin Cellobioselipide, Mannosylerythritollipide und Trehaloselipide.

Glycolipide haben nicht nur eine reinigende tensidische Wirkung sondern weisen auch eine konservierende Wirkung auf, wie in WO 2013/037977 A1 beschrieben. Zudem basieren sie auf nachwachsenden Rohstoffen. Biotenside und insbesondere Glycolipide sind daher besonders bevorzugte Tenside im Sinne der vorliegenden Erfindung.

Weist die Zusammensetzung eine Mischung unterschiedlicher Tenside auf, wobei eines der in dieser Tensidmischung enthaltenen Tenside ein Biotensid ist, so beträgt der Anteil an Biotensid und insbesondere Glycolipid in dieser Tensidmischung 20 Gew.-% bis 95 Gew.-%, insbesondere 35 Gew.-% bis 80 Gew.-%, bevorzugt 45 Gew.-% bis 70 Gew.-%.

Es ist besonders vorteilhaft, eine Kombination aus Aniontensid und Amphotensid, insbesondere einem Biotensid, einzusetzen. Diese Kombination bewirkt einerseits eine besonders gute Reinigungswirkung und kann zudem andererseits in Verbindung mit dem vorzugsweise eingesetzten Verdickungsmittel einen Beitrag zu einer räumlich besonders stabilen Suspension der Abrasivstoffteilchen leisten.

Das erfindungsgemäße Mittel kann zusätzlich ein oder mehrere nichtionische Tenside enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%.

Nichtionische Tenside im Rahmen der Erfindung können Alkoxylate sein wie Polyglycolether, Fettalkoholpolyglycolether, Alkylphenolpolyglycolether, endgruppenverschlossene Polyglycolether, Mischether und Hydroxymischether und Fettsäurepolyglycolester. Ebenfalls verwendbar sind Ethylenoxid-Propylenoxid-Blockpolymere, Fettsäurealkanolamide sowie Fettsäurepolyglycolether. Wichtige Klassen nichtionischer Tenside, die erfindungsgemäß verwendet werden können, sind weiterhin die Aminoxide sowie die Zuckertenside (Polyol-Tenside) und unter diesen besonders die Glykotenside, wie Alkylpolyglykoside und Fettsäureglucamide. Besonders bevorzugt sind die Alkylpolyglykoside, insbesondere die Alkylpolyglucoside.

Unter Fettalkoholpolyglykolethern sind erfindungsgemäß mit Ethylen- (EO) und/oder Pro-pylenoxid (PO) alkoxylierte, unverzweigte oder verzweigte, gesättigte oder ungesättigte C₁₀₋₂₂-Alkohole mit einem Alkoxylierungsgrad bis zu 30 zu verstehen, vorzugsweise ethoxylierte C₁₀₋₁₈-Fettalkohote mit einem Ethoxylierungsgrad von weniger als 30, bevorzugt mit einem Ethoxylierungsgrad von 1 bis 20, insbesondere von 1 bis 12, besonders bevorzugt von 1 bis 8, äußerst bevorzugt von 2 bis 5, beispielsweise C₁₂₋₁₄-Fettalkohol-ethoxylate mit 2, 3 oder 4 EO oder eine Mischung von der C₁₂₋₁₄-Fettalkoholethoxylate mit 3 und 4 EO im Gewichtsverhältnis von 1:1 oder Isotridecylalkoholethoxylat mit 5, 8 oder 12 EO.

Zu den erfindungsgemäß geeigneten Aminoxiden gehören Alkylaminoxide, insbesondere Alkyldimethylaminoxide, Alkylamidoaminoxide und Alkoxyalkylaminoxide. Bevorzugte Aminoxide genügen Formel (II) oder (IIb),

R⁶R⁷R⁸N⁺-O⁻ (IIa)

R⁶-[CO-NH-(CH₂)_{w}]_{z}-N⁺(R⁷)(R⁸)-O⁻ (IIb)

in der R⁶ ein gesättigter oder ungesättigter C₆₋₂₂-Alkylrest, vorzugsweise C₈₋₁₈-Alkylrest, insbesondere ein gesättigter C₁₀₋₁₆-Alkylrest, beispielsweise ein gesättigter C₁₂₋₁₄-Alkylrest, der in den Alkylamidoaminoxiden über eine Carbonylamidoalkylengruppe -CO-NH-(CH₂)_{z}- und in den Alkoxyalkylaminoxiden über eine Oxaalkylengruppe - O-(CH₂)_{z}- an das Stickstoffatom N gebunden ist, wobei z jeweils für eine Zahl von 1 bis 10, vorzugsweise 2 bis 5, insbesondere 3,
R⁷, R⁸ unabhängig voneinander ein C₁₄-Alkylrest, gegebenenfalls hydroxysubstituiert wie zum Beispiel ein Hydroxyethylrest, insbesondere ein Methylrest, ist.

Beispiele geeigneter Aminoxide sind die folgenden gemäß *INCI* benannten Verbindungen: Almondamidopropylamine Oxide, Babassuamidopropylamine Oxide, Behenamine Oxide, Cocamidopropyl Amine Oxide, Cocamidopropylamine Oxide, Cocamine Oxide, Coco-Morpholine Oxide, Decylamine Oxide, Decyltetradecylamine Oxide, Diaminopyrimidine Oxide, Dihydroxyethyl C₈₋₁₀-Alkoxypropylamine Oxide, Dihydroxyethyl C₉₋₁₁ Alkoxypropylamine Oxide, Dihydroxyethyl C₁₂₋₁₅-₋Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Hydrogenated Palm Kernel Amine Oxide, Hydrogenated Tallowamine Oxide, Hydroxyethyl Hydroxypropyl C₁₂₋₁₅-Alkoxypropylamine Oxide, Isostearamidopropylamine Oxide, Isostearamidopropyl Morpholine Oxide, Lauramidopropylamine Oxide, Lauramine Oxide, Methyl Morpholine Oxide, Milkamidopropyl Amine Oxide, Minkamidopropylamine Oxide, Myristamidopropylamine Oxide, Myristamine Oxide, Myristyl/Cetyl Amine Oxide, Oleamidopropylamine Oxide, Oleamine Oxide, Olivamidopropylamine Oxide, Palmitamidopropylamine Oxide, Palmitamine Oxide, PEG-3 Lauramine Oxide, Potassium Dihydroxyethyl Cocamine Oxide Phosphate, Potassium Trisphosphonomethylamine Oxide, Sesamidopropylamine Oxide, Soyamidopropylamine Oxide, Stearamidopropylamine Oxide, Stearamine Oxide, Tallowamidopropylamine Oxide, Tallowamine Oxide, Undecylenamidopropylamine Oxide und Wheat Germmidopropylamine Oxide. Ein bevorzugtes Aminoxid ist beispielsweise Cocamidopropylamine Oxide (Cocoamidopropylaminoxid).

Zuckertenside sind bekannte oberflächenaktive Verbindungen, zu denen beispielsweise die Zuckertensidklassen der Alkylglucoseester, Aldobionamide, Gluconamide (Zuckersäureamide), Glycerinamide, Glyceringlykolipide, Polyhydroxyfettsäureamidzuckertenside (Zuckeramide) und Alkylpolyglykoside zählen, wie sie etwa in der WO 97/00609 (Henkel Corporation*)* und den darin zitierten Druckschriften beschrieben sind (Seite 4 bis 12), auf die in dieser Hinsicht Bezug genommen und deren Inhalt hiermit in diese Anmeldung aufgenommen wird. Im Rahmen der erfindungsgemäßen Lehre bevorzugte Zuckertenside sind die Alkylpolyglykoside und die Zuckeramide sowie deren Derivate, insbesondere ihre Ether und Ester. Bei den Ethern handelt es sich um die Produkte der Reaktion einer oder mehrerer, vorzugsweise einer, Zuckerhydroxygruppe mit einer eine oder mehrere Hydroxygruppen enthaltenden Verbindung, beispielsweise C₁₋₂₂-Alkoholen oder Glykolen wie Ethylen- und/oder Propylenglykol, wobei die Zuckerhydroxygruppe auch Polyethylenglykol- und/oder Polypropylenglykolreste tragen kann. Die Ester sind die Reaktionsprodukte einer oder mehrerer, vorzugsweise einer, Zuckerhydroxygruppe mit einer Carbonsäure, insbesondere einer C₆₋₂₂-Fettsäure.

Besonders bevorzugte Zuckeramide genügen der Formel R'C(O)N(R")[Z], in der R' für einen linearen oder verzweigten, gesättigten oder ungesättigten Acylrest, vorzugsweise einen linearen ungesättigten Acylrest, mit 5 bis 21, vorzugsweise 5 bis 17, insbesondere 7 bis 15, besonders bevorzugt 7 bis 13 Kohlenstoffatomen, R" für einen linearen oder verzweigten, gesättigten oder ungesättigten Alkylrest, vorzugsweise einen linearen ungesättigten Alkylrest, mit 6 bis 22, vorzugsweise 6 bis 18, insbesondere 8 bis 16, besonders bevorzugt 8 bis 14 Kohlenstoffatomen, einen C₁₋₅-Alkylrest, insbesondere einen Methyl-, Ethyl-, Propyl-, Isopropyl-, n-Butyl-, Isobutyl-, tert-Butyl- oder n-Pentylrest, oder Wasserstoff und Z für einen Zuckerrest, das heißt einen Monosaccharidrest, stehen. Besonders bevorzugte Zuckeramide sind die Amide der Glucose, die Glucamide, beispielsweise Lauroyl-methyl-glucamid.

Die Alkylpolyglykoside (APG) sind im Rahmen der erfindungsgemäßen Lehre besonders bevorzugte Zuckertenside und genügen vorzugsweise der allgemeinen Formel R¹O(AO)ₐ[G]ₓ, in der R für einen linearen oder verzweigten, gesättigten oder ungesättigten Alkylrest mit 6 bis 22, vorzugsweise 6 bis 18, insbesondere 8 bis 16, besonders bevorzugt 8 bis 14 Kohlenstoffatomen, [G] für einen glykosidisch verknüpften Zuckerrest und x für eine Zahl von 1 bis 10 sowie AO für eine Alkylenoxygruppe, zum Beispiel eine Ethylenoxy- oder Propylenoxygruppe, und a für den mittleren Alkoxylierungsgrad von 0 bis 20 stehen. Hierbei kann die Gruppe (AO)ₐ auch verschiedene Alkylenoxyeinheiten enthalten, zum Beispiel Ethylenoxy- oder Propylenoxyeinheiten, wobei es sich dann bei a um den mittleren Gesamtalkoxylierungsgrad, das heißt die Summe aus Ethoxylierungs- und Propoxylierungsgrad, handelt. Soweit nachfolgend nicht näher beziehungsweise anders ausgeführt, handelt es sich bei den Alkylresten R¹ der APG um lineare ungesättigte Reste mit der angegebenen Zahl an Kohlenstoffatomen.

APG sind nichtionische Tenside und stellen bekannte Stoffe dar, die nach den einschlägigen Verfahren der präparativen organischen Chemie erhalten werden können. Die Indexzahl *x* gibt den Oligomerisierungsgrad (DP-Grad) an, das heißt die Verteilung von Mono- und Oligoglykosiden, und steht für eine Zahl zwischen 1 und 10. Während *x* in einer gegebenen Verbindung stets ganzzahlig sein muss und hier vor allem die Werte *x* = 1 bis 6 annehmen kann, ist der Wert *x* für ein bestimmtes Alkylglykosid eine analytisch ermittelte rechnerische Größe, die meistens eine gebrochene Zahl darstellt. Vorzugsweise werden Alkylglykoside mit einem mittleren Oligomerisierungsgrad *x* von 1,1 bis 3,0 eingesetzt. Aus anwendungstechnischer Sicht sind solche Alkylglykoside bevorzugt, deren Oligomerisierungsgrad kleiner als 1,7 ist und insbesondere zwischen 1,2 und 1,6 liegt. Als glykosidischer Zucker wird vorzugsweise Xylose, insbesondere aber Glucose verwendet.

Der Alkyl- beziehungsweise Alkenylrest R¹ kann sich von primären Alkoholen mit 8 bis 18, vorzugsweise 8 bis 14 Kohlenstoffatomen ableiten. Typische Beispiele sind Capronalkohol, Caprylalkohol, Caprinalkohol und Undecylalkohol sowie deren technische Gemische, wie sie beispielsweise im Verlauf der Hydrierung von technischen Fettsäuremethylestern oder im Verlauf der Hydrierung von Aldehyden aus der ROELENschen Oxosynthese anfallen.

Vorzugsweise leitet sich der Alkyl- beziehungsweise Alkenylrest R¹ aber von Laurylalkohol, Myristylalkohol, Cetylalkohol, Palmoleylalkohol, Stearylalkohol, Isostearylalkohol oder Oleylalkohol ab. Weiterhin sind Elaidylalkohol, Petroselinylalkohol, Arachidylalkohol, Gadoleylalkohol, Behenylalkohol, Erucylalkohol sowie deren technische Gemische zu nennen.

Besonders bevorzugte APG sind nicht alkoxyliert (a = 0) und genügen Formel RO[G]ₓ in der R wie zuvor für einen linearen oder verzweigten, gesättigten oder ungesättigten Alkylrest mit 4 bis 22 Kohlenstoffatomen, [G] für einen glykosidisch verknüpften Zuckerrest, vorzugsweise Glucoserest, und *x* für eine Zahl von 1 bis 10, bevorzugt 1,1 bis 3, insbesondere 1,2 bis 1,6, stehen. Dementsprechend bevorzugte Alkylpolyglykoside sind beispielsweise C₈₋₁₀- und ein C₁₂₋₁₄-Alkylpolyglucosid mit einem DP-Grad von 1,4 oder 1,5, insbesondere C₈₋₁₀- Alkyl-1,6-glucosid und C₁₂₋₁₄-Alkyl-1,4-glucosid.

Das erfindungsgemäße Mittel kann zusätzlich ein oder mehrere kationische Tenside (Kationtenside; *INCI* Quaternary Ammonium Compounds) enthalten, üblicherweise in einer Menge von 0,001 bis 5 Gew.-%, vorzugsweise 0,01 bis 4 Gew.-%, insbesondere 0,1 bis 3 Gew.-%, besonders bevorzugt 0,2 bis 2 Gew.-%, äußerst bevorzugt 0,5 bis 1,5 Gew.-%, beispielsweise 1 Gew.-%. In einer bevorzugten Ausführungsform wird jedoch auf den Einsatz kationischer Tenside verzichtet.

Bevorzugte kationische Tenside sind die quaternären oberflächenaktiven Verbindungen, insbesondere mit einer Ammonium-, Sulfonium-, Phosphonium-, Jodonium- oder Arsoniumgruppe, wie sie beispielsweise K. *H. Wallhäußer* in "Praxis der Sterilisation, Desinfektion - Konservierung : Keimidentifizierung - Betriebshygiene" (5. Aufl. - Stuttgart; New York: Thieme, 1995) als antimikrobielle Wirkstoffe beschreibt. Durch den Einsatz von quaternären oberflächenaktiven Verbindungen mit antimikrobieller Wirkung kann das Mittel mit einer antimikrobiellen Wirkung ausgestaltet werden beziehungsweise dessen gegebenenfalls aufgrund anderer Inhaltsstoffe bereits vorhandene antimikrobielle Wirkung verbessert werden.

Besonders bevorzugte kationische Tenside sind zusätzlich zu den als Trocknungs- und Glanzadditiven verwendeten quartären Ammoniumverbindungen die quaternären, zum Teil antimikrobiell wirkenden Ammoniumverbindungen (QAV; *INCI* Quaternary Ammonium Compounds) gemäß der allgemeinen Formel (R^{I})(R^{II})(R^{III})(R^{IV})N⁺ X⁻, in der R^{I} bis R^{IV} gleiche oder verschiedene C₁₋₂₂-Alkylreste, C₇₋₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder Imidazoliniumverbindung, bilden, darstellen und X⁻Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere 12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzyl-ammoniumchlorid, CAS No. 8001-54-5), Benzalkon B (*m,p*-Dichlorbenzyl-dimethyl-C₁₂-alkylammoniumchlorid, CAS No. 58390-78-6), Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, CAS No. 57-09-0), Benzetoniumchlorid (N,N-Dimethyl-N-[2-[2[p- (1,1,3,3-tetramethylbutyl)phenoxy]ethoxy]ethyl]-benzylammoniumchlorid,CAS No.121-54-0), Dialkyldimethylammoniumchloride wie Di-n-decyldimethylammoniumchlorid (CAS No. 7173-51-5-5), Didecyldimethylammoniumbromid (CAS No. 2390-68-3), Dioctyldimethylammoniumchloric, 1-Cetylpyridiniumchlorid (CAS No. 123-03-5) und Thiazolinjodid (CAS No. 15764-48-1) sowie deren Mischungen. Bevorzugte QAV sind die Benzalkoniumchloride mit C₈-C₁₈-Alkylresten, insbesondere C₁₂-C₁₄-Alkylbenzyldimethylammoniumchlorid. Eine besonders bevorzugte QAV Kokospentaethoxymethylammoniummethosulfat *(INCI* PEG-5 Cocomonium Methosulfate; Rewoquat^{®} CPEM).

Zur Vermeidung möglicher Inkompatibilitäten der antimikrobiellen kationischen Tenside mit den erfindungsgemäß enthaltenen anionischen Tensiden werden möglichst aniontensidverträgliches und/oder möglichst wenig kationisches Tensid eingesetzt oder in einer besonderen Ausführungsform der Erfindung gänzlich auf antimikrobiell wirkende kationische Tenside verzichtet. Als antimikrobiell wirksame Substanzen können Parabene, Benzoesäure und/oder Benzoat, Milchsäure und/oder Lactate eingesetzt werden. Besonders bevorzugt sind Benzoesäure und/oder Milchsäure. Besonders bevorzugt weist die Zusammensetzung Salze von Alkylethersulfaten, Alkylbetaine und Alkylaminoxide auf.

Die Zusammensetzung kann weiterhin wenigstens ein Lösungsmittel, Verdicker und/oder Salze aufweisen. Die Zusammensetzung kann flüssig sein. Insbesondere wenn sie ein Reinigungsmittel zur manuellen Reinigung von Geschirr ist, ist sie flüssig und zudem transparent. In diesem Fall unterscheiden sich das natürliche Material sowie die Zusammensetzung an sich in ihrer Farbe, sodass eine transparente Flüssigkeit erhalten wird, in welcher weißliche oder opake Teilchen gleichmäßig dispergiert sind. Um eine gleichmäßige Verteilung des natürlichen Materials in der Zusammensetzung zu ermöglichen, weist dies vorzugsweise eine Fließgrenze auf. Eine Fließgrenze kann dabei durch die Verwendung von Verdickern, insbesondere polymerer Verdicker, und/oder Salze ermöglicht werden. Ist die Zusammensetzung flüssig, weist sie insbesondere eine Fließgrenze bei leichter bis mittlerer Scherung auf, dass heißt das Speichermodul G' des Systems ist bei Scherraten von 0,0001 bis 0,1 s⁻¹ größer als das Verlustmodul G".

Unter der Fließgrenze wird somit in der Rheologie diejenige Schubspannung (in Pa) verstanden unterhalb derer sich eine Probe ausschließlich oder zumindest weitgehend elastisch verformt, und oberhalb derer eine irreversible, plastische Verformung, also ein Fließen stattfindet. Eine solche Fließgrenze wird üblicherweise durch Messung mit einem Rheometer bei Raumtemperatur, also einer Temperatur von 20 °C bis 25 °C, insbesondere von 25 °C, bestimmt. Absolut messende Rheometer, wie beispielsweise das Rheometer AR 1000-N der Firma Texas Instruments ermöglichen die Ermittlung von der Messgeometrie unabhängigen und verlässlichen, absoluten Messwerten.

Die Fließgrenze der flüssigen, Tensid-enthaltenden Zusammensetzung kann erfindungsgemäß beispielsweise auch mit einem Rotationsrheometer der Firma TA-Instruments, Typ AR G2 (schubspannungskontrolliertes Rheometer, Kegel-Platte Messsystem mit 40 mm Durchmesser, 2° Kegelwinkel, 20°C) gemessen werden. Hierbei handelt es sich um ein so genanntes schubspannungskontrolliertes Rheometer. Hier werden die Proben im Rheometer mit einer mit der Zeit ansteigenden Schubspannung *σ* (t) beaufschlagt. Beispielsweise kann die Schubspannung im Laufe von 30 Minuten vom kleinstmöglichen Wert (zum Beispiel 0,01 Pa) auf zum Beispiel 100 Pa gesteigert werden. Als Funktion dieser Schubspannung *σ* wird die Deformation y der Probe gemessen. Die Deformation wird in einem doppellogarithmischen Plot gegen die Schubspannung aufgetragen (log *γ* gegen log *σ*). Sofern die untersuchte Probe eine Fließgrenze aufweist, kann man diese durch eine sprunghafte Änderung der Kurve erkennen. Unterhalb einer gewissen Schubspannung findet man eine rein elastische Deformation. Die Steigung der Kurve *γ*(*σ*) (log-log-Plot) in diesem Bereich ist eins. Oberhalb dieser Schubspannung beginnt viskoses Fließen und die Steigung der Kurve ist sprunghaft höher. Diejenige Schubspannung, bei der das Abknicken der Kurve erfolgt, also der Übergang von der elastischen in eine plastische Deformation, markiert die Fließgrenze. Eine bequeme Bestimmung der Fließgrenze (= Knick der Kurve) ist durch Anlegen von Tangenten an die beiden Kurventeile möglich. Proben ohne Fließgrenze weisen keinen charakteristischen Knick in der Funktion *γ*(*σ*) auf.

Die erfindungsgemäße Zusammensetzung weist bevorzugt eine Fließgrenze im Bereich von 0,01 Pa bis 10 Pa, bevorzugt von 0,1 Pa bis 5 Pa, besonders bevorzugt von 0,5 Pa bis 3 Pa auf.

Entsprechend flüssige Zusammensetzungen mit Fließgrenze sind im Stand der Technik hinlänglich beschrieben.

Vorzugsweise weist die Zusammensetzung wenigstens einen Verdicker in einem Anteil von 0,001 bis 5 Gew.-%, insbesondere von 0,005 bis 3,5 Gew.-%, bevorzugt von 0,01 bis 2 Gew.-% auf.

In einer bevorzugten Ausführungsform enthält das erfindungsgemäße Handgeschirrspülmittel zur Absenkung der Viskosität weiterhin ein oder mehrere wasserlösliche Salze. Es kann sich dabei um anorganische und/oder organische Salze handeln, in einer bevorzugten Ausführungsform enthält das Mittel dabei mindestens ein anorganisches Salz.

Erfindungsgemäß einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben.

Bei den erfindungsgemäß einsetzbaren organischen Salzen handelt es sich insbesondere um farblose wasserlösliche Alkalimetall-, Erdalkalimetall-, Ammonium-, Aluminium- und/oder Übergangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Das erfindungsgemäße Handgeschirrspülmittel enthält in einer bevorzugten Ausführungsform 0,1 bis 10 Gew.-%, vorzugsweise 0,5 bis 7 Gew.-%, besonders bevorzugt 0,8 bis 5 Gew.-% mindestens eines wasserlöslichen Salzes. In einer besonders bevorzugten Ausführungsform werden dabei ausschließlich anorganische Salze eingesetzt.

Verdickungsmittel im Sinne der vorliegenden Erfindung sind Polycarboxylate, vorzugsweise Homo- und Copolymerisate der Acrylsäure, insbesondere Acrylsäure-Copolymere wie Acrylsäure-Methacrylsäure-Copolymere, und Polysaccharide, insbesondere Heteropolysaccharide, sowie andere übliche polymere Verdicker. Daneben können auch Schichtsilikate und weitere dem Fachmann als Verdickungsmittel bekannte anorganische Stoffe als Verdickungsmittel im Sinne dieser Erfindung eingesetzt werden; auch Gemische aus verschiedenen Verdickungsmitteln können zum Einsatz kommen. Es ist lediglich darauf zu achten, dass beim Einsatz des Verdickungsmittels der weitgehend transparente optische Eindruck erhalten bleibt. Vorzugsweise werden jedoch Polymere, vor allem Polycarboxylate und/oder Polysaccharide, als Verdickungsmittel in erfindungsgemäßen Reinigungsmitteln eingesetzt.

Geeignete Polysaccharide beziehungsweise Heteropolysaccharide sind die Polysaccharidgummen, beispielsweise Gummi arabicum, Agar, Alginate, Carrageene und ihre Salze, Guar, Guaran, Tragacant, Gellan, Ramsan, Dextran oder Xanthan und ihre Derivate, zum Beispiel propoxyliertes Guar, sowie ihre Mischungen. Andere Polysaccharidverdicker, wie Stärken oder Cellulosederivate, können alternativ, vorzugsweise aber zusätzlich zu einem Polysaccharidgummi eingesetzt werden, beispielsweise Stärken verschiedensten Ursprungs und Stärkederivate, zum Beispiel Hydroxyethylstärke, Stärkephosphatester oder Stärkeacetate, oder Carboxymethylcellulose beziehungsweise ihr Natriumsalz, Methyl-, Ethyl-, Hydroxyethyl-, Hydroxypropyl-, Hydroxypropylmethyl- oder Hydroxyethymethylcellulose oder Celluloseacetat.

Ein bevorzugtes Polymer ist das mikrobielle anionische Heteropolysaccharid Xanthan Gum, das von Xanthomonas campestris und einigen anderen Spezies unter aeroben Bedingungen mit einem Molekulargewicht von 2 bis 15×10⁶ produziert wird und beispielsweise von der Fa. Kelco unter den Handelsnamen *Keltrof*^{®} und *Kelzan*^{®} oder auch von der Firma Rhodia unter dem Handelsnamen *Rhodopol*^{®} erhältlich ist.

Ein weiteres bevorzugtes Polymer ist das ebenfalls mikrobielle Heteropolysaccharid Gellan Gum, das unter aeroben Bedingungen beispielsweise von *Auromonas elodea* und vor allem von *Sphingomonas paucimobilis-* Stämmen gebildet wird. Gellan Gum kann beispielsweise unter dem Handelsnamen *Kelcogel*^{®} in verschiedenen Qualitäten von der Firma *Kelco* bezogen werden.

Geeignete Acrylsäure-Polymere sind beispielsweise hochmolekulare mit einem Polyalkenylpolyether, insbesondere einem Allylether von Saccharose, Pentaerythrit oder Propylen, vernetzte Homopolymere der Acrylsäure *(INCI* Carbomer), die auch als Carboxyvinylpolymere bezeichnet werden. Solche Polyacrylsäuren sind unter anderem von der Fa. *BFGoodrich* unter dem Handelsnamen *Carbopof*^{®} erhältlich.

Besonders geeignete Polymere sind aber folgende Acrylsäure-Copolymere: (i) Copolymere von zwei oder mehr Monomeren aus der Gruppe der Acrylsäure, Methacrylsäure und ihrer einfachen, vorzugsweise mit C₁₄-Alkanolen gebildeten, Ester *(INCI* Acrylates Copolymer), zu denen etwa die Copolymere von Methacrylsäure, Butylacrylat und Methylmethacrylat (CAS 25035-69-2) oder von Butylacrylat und Methylmethacrylat (CAS 25852-37-3) gehören und die beispielsweise von der Fa. *Rohm* & Haas unter den Handelsnamen *Aculyn*^{®} und *Acusol*^{®} sowie von der Firma *Degussa (Goldschmidt)* unter dem Handelsnamen *Tego*^{®} *Polymer* erhältlich sind; (ii) vernetzte hochmolekulare Acrylsäurecopolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₄-Alkanolen gebildeten, Ester *(INCI* Acrylates/C10-30 Alkyl Acrylate Crosspolymer) gehören und die beispielsweise von der Fa. *BFGoodrich* unter dem Handelsnamen *Carbopol*^{®} erhältlich sind. Geeignete Acrylsäureester sind ebenfalls unter dem Handelsnamen Skalan^{®}AT 120 und Rheovis^{®} AT 120 von der Firma BASF erhältlich. Werden Acrylsäurepolymere und insbesondere Acrylsäureester als polymere Verdicker eingesetzt, so beträgt der pH-Wert vorzugsweise mehr als 7, insbesondere wenigstens 7,5, bevorzugt 8 oder mehr.

Als anorganische Verdickungsmittel können vor allem Schichtsilikate eingesetzt werden. Hierzu zählen beispielsweise die unter dem Handelsnamen *Laponite*^{®} erhältlichen Magnesium- oder Natrium-Magnesium- Schichtsilikate der Firma *Solvay Alkali,* insbesondere das *Laponite*^{®} RD oder auch *Laponite*^{®} *RDS,* sowie die Magnesiumsilikate der Firma *Süd-Chemie,* vor allem das *Optigel*^{®} *SH.*

Die Auswahl des Verdickungsmittels erfolgt immer auch mit der Maßgabe, dass der weitgehend transparente optische Eindruck erhalten bleiben soll, das heißt Eintrübungen durch das Verdickungsmittels sind weitgehend zu vermeiden Der Gehalt an Verdickungsmittel beträgt üblicherweise zwischen 0,01 und 8 Gew.-%, vorzugsweise zwischen 0,1 und 6 Gew.-%, besonders bevorzugt zwischen 0,5 und 3 Gew.-%, beispielsweise zwischen 0,5 und 1 Gew.-% oder zwischen 2 und 3 Gew.-%. Die Viskosität der erfindungsgemäßen Mittel wird wesentlich über den Gehalt an Verdickungsmittel eingestellt beziehungsweise gesteuert, wobei die erforderlichen Mengen von Verdicker zu Verdicker unterschiedlich sein können. Auch die verwendete Tensidzusammensetzung spielt in der Mengenwahl eine Rolle.

Je nach eingesetztem Verdickungsmittel kann es wünschenswert sein, zur Stabilisierung zusätzlich Elektrolytsalze einzusetzen. Elektrolytsalze im Sinne dieser Erfindung sind dabei Salze aus vorzugsweise mehrwertigen Kationen mit anorganischen Säureresten. Insbesondere bevorzugt sind hierbei die Chloride und Sulfate der Erdalkalimetalle, des Aluminiums und des Zinks, vor allem das Aluminiumchlorid. Der Einsatz dieser Elektrolytsalze ist vor allem in Verbindung mit Polysaccharid-Verdickungsmitteln wie Xanthan oder Gellan von Vorteil.

Es ist jedoch auch möglich, dass die Zusammensetzung fest, beispielsweise pastös ist. Eine Fließgrenze ist in einem solchen Fall nicht unbedingt notwendig.

Vorzugsweise weist die Zusammensetzung, wenn sie flüssig ist, Wasser als Lösungsmittel auf, der Anteil an Wasser beträgt insbesondere 1 Gew.-% bis 99 Gew.-%, vorzugsweise 95 Gew.-% oder weniger, insbesondere 90 Gew.-% oder weniger, bevorzugt 5 Gew.-% oder 10 Gew.-% oder mehr. Darüber hinaus kann eine flüssige Zusammensetzung noch weitere Lösungsmittel, wie beispielsweise kurzkettige Alkohole aufweisen. Zudem sind üblicherweise auch eine oder mehrere Hilfs- oder Zusatzstoffe enthalten, wie sie im Stand der Technik hinlänglich beschrieben sind. Diese sind vorzugsweise ausgewählt aus Enzymen, UV-Stabilisatoren, Parfümstoffen, Farbstoffen, Polymeren, Reinigungsverstärkern, Desinfektionsmitteln, Antistatika, Korrosionsinhibitoren, Konservierungsmitteln, Hautschutzmitteln, sowie Mischungen dieser.

### Beispiel 1:

Es wurden flüssige Reinigungsmittel-Rezepturen (Handgeschirrspülmittel) entwickelt.

Die Angaben erfolgen in Gew.-%. Das Gesamtgewicht entsprach 100 Gew.-%.

V1 und E1 bis E4 sind Vergleichsbeispiele.

| | **E1** | **E2** | **E3** | **E4** | **V1** |
|---|---|---|---|---|---|
| expandiertes Vulkanglas* | 0,2 | 0,4 | 1,0 | 1,5 | 0 |
| Anionisches Tensid (Alkylethersulfas 2 EO, Na-Salz (C₁₂₋14)) | 10,0 | 12,0 | 14,0 | 16,0 | 14,0 |
| Amphotensid (Cocamidopropylbetain) | 4,0 | 4,0 | 4,0 | 4,0 | 4,0 |
| Kationisches Tensid (Alkylaminoxid (C₁₂)) | 3,0 | 3,0 | 3,0 | 3,0 | 3,0 |
| NaCl | 2,0 | 2,0 | 2,0 | 2,0 | 2,0 |
| Polymerverdicker** | 0,2 | 0,25 | 0,3 | 0,35 | 0,3 |
| Parfüm | 0,8 | 0,8 | 0,8 | 0,8 | 0,8 |
| Ethanol | 1,5 | 1,5 | 1,5 | 1,5 | 1,5 |
| Wasser | Rest | Rest | Rest | Rest | Rest |

| | | | | | |
|---|---|---|---|---|---|
| * Perlite von der Fa. Imerys ** Sokalan^{®} AT 120 der Fa. BASF | | | | | |

### Beispiel 2:

### Reinigungswirkung:

Es wurde die Rezeptur E3 getestet gegen die gleiche Rezeptur ohne den Zusatz von expandiertem Vulkanglas (V1). Als Anschmutzung wurde eine Ragu Sauce verwendet, welche auf einer Stahlplatte 2 h bei 160 °C eingebrannt wurde. Die Rezepturen wurden mit 5 ml direkt auf die Anschmutzung dosiert. Danach wird mit einem Mehrspurwischgerät mit Tuchhalter (Sheen 903 PG) die Entfernung analog der IKW Empfehlung zur Qualitätsbewertung der Produktleistung von Allzweckreinigern 2014 (SÖFW Journal 2014, S. 47) getestet. Als Tücher wurden solche der Artikelnummer 02010100 der Fa. Wecovi verwendet.

Die Entfernung wurde optisch von einem Panel von geschulten Experten auf einer Skala von 1 bis 5 (5 = vollständige Entfernung) bewertet. Ergebnis:

| | |
|---|---|
| Reinigungsleistung E3: | 3,7 |
| Reinigungsleistung Vergleichsrezeptur V1: | 3,2 |

## Patentansprüche

1. Zusammensetzung umfassend wenigstens ein Tensid und wenigstens ein natürliches Material mit einer Mohs-Härte von 4 bis 8, insbesondere von 5 bis 6, **dadurch gekennzeichnet, dass**
das natürliche Material in Hydrogelpartikeln eingebettet vorliegt;
das natürliche Material Bimsstein ist; und
die Zusammensetzung eine Zusammensetzung zur Reinigung von harten Oberflächen ist.

2. Zusammensetzung nach Anspruch 1, **dadurch gekennzeichnet, dass** das natürliche Material eine Teilchengröße im Bereich von 0,1 µm bis 50.000 µm, insbesondere von 1 µm bis 1.000 µm aufweist.

3. Zusammensetzung nach einem der Ansprüche 1 bis 2, **dadurch gekennzeichnet, dass** das natürliche Material einen Partikelformfaktor von 0,1 bis 0,97, insbesondere von 0,15 bis 0,7 aufweist.

4. Zusammensetzung nach einem der Ansprüche 1 bis 3, **dadurch gekennzeichnet, dass** das wenigstens eine Tensid in einem Anteil von 5 Gew.-% bis 35 Gew.-%, insbesondere von 8 Gew.-% bis 25 Gew.-% enthalten ist.

5. Zusammensetzung nach einem der Ansprüche 1 bis 4, **dadurch gekennzeichnet, dass** das wenigstens eine natürliche Material in einem Anteil von 0,02 Gew.-% bis 40 Gew.-%, insbesondere von 0,05 Gew.-% bis 10 Gew.-%, besonders von 0,2 Gew.-% bis 5 Gew.-% enthalten ist.

6. Zusammensetzung nach einem der Ansprüche 1 bis 5, **dadurch gekennzeichnet, dass** sie weiterhin wenigstens ein Lösungsmittel, Verdicker und/oder Salze aufweist.

7. Zusammensetzung nach Anspruch 6, **dadurch gekennzeichnet, dass** der Verdicker ein Acrylsäurepolymer umfasst.

8. Zusammensetzung nach einem der Ansprüche 1 bis 7, **dadurch gekennzeichnet, dass** wenigstens ein Aniontensid und/oder wenigstens ein nichtionisches Tensid und/oder wenigstens ein amphoteres Tensid, bevorzugt ein Aniontensid und ein nichtionisches Tensid und ein amphoteres Tensid aufweist.

9. Zusammensetzung nach Anspruch 8, weiterhin umfassend einen oder mehrere Hilfs- oder Zusatzstoffe, ausgewählt aus Enzymen, UV-Stabilisatoren, Parfümstoffen, Farbstoffen, Polymeren, Reinigungsverstärkern, Desinfektionsmitteln, Antistatika, Korrosionsinhibitoren, Konservierungsmitteln, Hautschutzmitteln, sowie Mischungen dieser.

## Claims

1. Composition comprising at least one surfactant and at least one natural material with a Mohs hardness of from 4 to 8, in particular 5 to 6, **characterized in that**
the natural material is embedded in hydrogel particles;
the natural material is pumice; and
the composition is a composition for cleaning hard surfaces.

2. Composition according to claim 1, **characterized in that** the natural material has a particle size in the range from 0.1 µm to 50,000 µm, in particular from 1 µm to 1000 µm.

3. Composition according to either of claims 1 to 2, **characterized in that** the natural material has a particle shape factor of from 0.1 to 0.97, in particular 0.15 to 0.7.

4. Composition according to any of claims 1 to 3, **characterized in that** the at least one surfactant is present in a proportion of from 5 wt.% to 35 wt.%, in particular from 8 wt.% to 25 wt.%.

5. Composition according to any of claims 1 to 4, **characterized in that** the at least one natural material is present in a proportion of from 0.02 wt.% to 40 wt.%, in particular from 0.05 wt.% to 10 wt.%, specifically from 0.2 wt.% to 5 wt.%.

6. Composition according to any of claims 1 to 5, **characterized in that** it also comprises at least one solvent, thickener, and/or salts.

7. Composition according to claim 6, **characterized in that** the thickener comprises an acrylic acid polymer.

8. Composition according to any of claims 1 to 7, **characterized in that** it contains at least one anionic surfactant and/or at least one non-ionic surfactant and/or at least one amphoteric surfactant, preferably one anionic surfactant, one non-ionic surfactant, and one amphoteric surfactant.

9. Composition according to claim 8, further comprising one or more auxiliaries or additives selected from enzymes, UV stabilizers, perfumes, dyes, polymers, cleaning boosters, disinfectants, antistatic agents, corrosion inhibitors, preservatives, skin protection agents, and mixtures thereof.

## Revendications

1. Composition comprenant au moins un tensioactif et au moins une matière naturelle présentant une dureté Mohs de 4 à 8, en particulier de 5 à 6, **caractérisée en ce que**
la matière naturelle est incorporée dans des particules d'hydrogel ;
la matière naturelle est la pierre ponce ; et
la composition est une composition destinée au nettoyage des surfaces dures.

2. Composition selon la revendication 1, **caractérisée en ce que** la matière naturelle présente une granulométrie dans la plage comprise entre 0,1 µm et 50 000 µm, en particulier entre 1 µm et 1 000 µm.

3. Composition selon l'une des revendications 1 à 2, **caractérisée en ce que** la matière naturelle présente un facteur de forme des particules de 0,1 à 0,97, en particulier de 0,15 à 0,7.

4. Composition selon l'une quelconque des revendications 1 à 3, **caractérisée en ce que** l'au moins un tensioactif est contenu en une proportion de 5 % en poids à 35 % en poids, en particulier de 8 % en poids à 25 % en poids.

5. Composition selon l'une quelconque des revendications 1 à 4, **caractérisée en ce que** l'au moins une matière naturelle est contenue en une proportion de 0,02 % en poids à 40 % en poids, en particulier de 0,05 % en poids à 10 % en poids, de manière particulièrement préférée de 0,2 % en poids à 5 % en poids.

6. Composition selon l'une quelconque des revendications 1 à 5, **caractérisée en ce qu'**elle comporte en outre au moins un solvant, un épaississant et/ou des sels.

7. Composition selon la revendication 6, **caractérisée en ce que** l'épaississant comprend un polymère d'acide acrylique.

8. Composition selon l'une quelconque des revendications 1 à 7, **caractérisée en ce qu'**elle comporte au moins un tensioactif anionique et/ou au moins un tensioactif non ionique et/ou au moins un tensioactif amphotère, de préférence un tensioactif anionique et un tensioactif non ionique et un tensioactif amphotère.

9. Composition selon la revendication 8, comprenant en outre au moins un auxiliaire ou additif choisis parmi les enzymes, les stabilisants UV, les parfums, les colorants, les polymères, les renforçateurs de nettoyage, les désinfectants, les agents antistatiques, les inhibiteurs de corrosion, les conservateurs, les agents de protection de la peau et leurs mélanges.
